# EUROPEAN PATENT APPLICATION

(11) **EP 2 937 051 A1**
(43) Date of publication of application: **28.10.2015**
(21) Application number: 14166075.3
(22) Date of filing: 25.04.2014
(51) Int. Cl.: A61B 17/80

(54) **Implant for tibial plateau leveling osteotomy fixation**

(71) Applicant: Kyon AG, 8005 Zürich (CH)
(72) Inventor: Tepic, Slobodan, 8006 Zurich (CH); Stearns, Phillips, South Hamilton, MA Massachusetts 01982 (US)
(74) Representative: Weickmann & Weickmann

(57) **Abstract**

A method and implant to treat anterior cruciate ligament (ACL) injuries are disclosed. The method involves performing the cylindrical osteotomy of the proximal tibia and rotating the tibia plateau to a position of about 90 degrees to the patellar tendon with the stifle extended. The grate-like implant of the same shape and the thickness as the saw used to perform the osteotomy is specifically designed to facilitate transfer of compressive loads between the proximal and distal tibia segments. The implant also comprises a flange that is fixed by screws to the bone segments to retain the position of the grate-like spacer within the osteotomy gap and also to maintain the relative position of the bone segments after the leveling of the tibia plateau.

## Description

### BACKGROUND OF THE INVENTION

### 1. Field of the Invention

The invention relates to a method and implants to treat a knee with an injured or ruptured anterior cruciate ligament.

### 2. Discussion of Related Art

The Anterior Cruciate Ligament (ACL) in the human knee joint, commonly called Cranial Cruciate Ligament (CrCL) in the canine stifle, is frequently torn in trauma. It also frequently fails, particularly in dogs, after a degenerative process of still unknown etiology.

In human orthopedics, the standard procedure calls for replacement by an ACL allograft, a part of the patient's own patellar tendon, or a part of the tendon removed from the hamstring muscle. The procedure results in a stable knee, but the long-term performance is often unsatisfactory -- 75 to 90% of cases result in arthrosis of the joint within 15 years of the procedure.

In dogs, the standard procedure is either an extra capsular suture or one of several geometry modifying surgical techniques. In the extra capsular procedure, a suture is placed outside of the joint, usually on the lateral side, to approximate the function of the ligament. The intention of placing this suture is to provide stability of the joint for several weeks, while waiting for fibrosis to form around the joint. The fibrosis should provide for long-term stability. However, the extra capsular suture technique regularly results in failure. Arthrosis of the joint, at a year or so, is the rule rather exception.

In surgical techniques, the tibia is cut and a segment of it is repositioned to change the geometry in order to stabilize the stifle. Various techniques have been used including: tibial plateau leveling osteotomy (TPLO; Slocum B, Slocum TD; Tibial Plateau Leveling Osteotomy For Repair Of Cranial Cruciate Ligament Rupture In The Canine, Vet. Clin. North Am. 23: 777-795, 1993), cranial closing wedge osteotomy (CWO; Slocum B, Devine T; Cranial Tibial Wedge Osteotomy: A Technique For Eliminating Cranial Tibial Thrust In Cranial Cruciate Ligament Repair, J. Am. Vet. Med. Assoc. 184: 564-569, 1984), and tibial tuberosity advancement (TTA; Tepic S, Damur DM, Montavon PM; Biomechanics Of The Stifle Joint, in Proceedings of the 1st World Orthopaedic Vet. Congress, Munich, Germany, Sept 2002, pp 189-190). Surgical complications are not uncommon with each of these techniques.

Intra-articular prostheses are also occasionally used in dogs and, currently, only on an experimental basis in humans. Again, they require significantly invasive procedures to insert them within the stifle. Even then, they generally end up in failure.

### SUMMARY OF THE INVENTION

This invention provides a solution for a less invasive, yet more robust stabilization of a TPLO, with implants specifically addressing the liabilities of the procedure related to compromised bony support on the lateral aspect of the tibia. According to one aspect of the invention, a grate-like, thin, cylindrically shaped spacer is configured to be placed into the osteotomy, between the proximal and the distal segments of the tibia. The implant is sized and shaped to provide inter-segmental support for the shifted and thus miss-matched cortices at the level of the osteotomy. According to another aspect of the invention, the implant is made from an artificial material, e.g. metal (titanium, a titanium alloy, stainless steel or a cobalt chromium alloy), highly perforated to allow bony through-growth and surface-treated for optimum bony tissue tolerance and adhesion. According to another aspect of the invention, the spacer is provided by flange-like means to allow for its retention within the osteotomy, by for example screws inserted through the flange into the tibia from the medial aspect. According to another aspect of the invention, the surfaces of the grate-like implant facing the bone are treated to result in high friction or mechanical interlock and to favor intimate bone apposition or ingrowth. The material for the grate should in all circumstances be biocompatible.

According to another aspect of the invention, the implant, with its flange-like component and the screws provide stabilization of the osteotomy in the saggital plane as well as resisting the tensile forces on the medial side of the tibia.

The procedure includes rotating the proximal segment of the tibia so as to decrease the plateau slope by a predetermined angle, according to the teachings of Slocum and securing the new position by inserting the screws of the flange. A minimum of two screws in one segment and one screw in the other segment are to be used. For a better balance of stresses, use of two screws in each segment is recommended.

### BRIEF DESCRIPTION OF THE DRAWINGS

Figure 1 is a side view of a dog knee joint (stifle) on which the TPLO osteotomy has been performed.
Figure 2 is a side view of a dog knee joint having a tibial plateau rotated and the new position fixed by conventional TPLO plating.
Figure 3 is a cross section of the tibia along the TPLO osteotomy.
Figure 4 shows cross sections of the tibia along the TPLO osteotomy after the proximal segment has been rotated to level the plateau.
Figure 5 is a posterior view of the proximal tibia with conventional TPLO plating.
Figure 6 shows a side view of a TPLO stabilized with implants according to the invention.
Figure 7 shows a grate-like implant according to the invention cut from a sheet metal prior to bending into cylindrical shape.
Figure 8 shows the flange of the implant according to the invention.
Figure 9 is a perspective view of the implant according to the invention.

### DETAILED DESCRIPTION

In Tibial Plateau Leveling Osteotomy (TPLO), two segments of a bone (tibia) are produced by a curvilinear cut. The present invention provides a grate-like implant to be used to fix the proximal and distal segments of the tibia following TPLO and the rotation of the proximal segment aimed at eliminating the shear force in the stifle with a damaged or ruptured cranial cruciate ligament. The main rationale of the implant is to provide a better support for the load transfer between the cortices of the proximal and distal segments, which after the relative rotation are seriously mismatched in shape. A rather common problem with the state of the art technique to fix the segments, which is with a plate and bone screws placed on the medial cortex, is a collapse of the lateral side. This is due to greatly reduced area of contact on the lateral aspect. The plates placed on the medial cortex do not have the strength and stiffness to provide enough support the lateral side of the osteotomy.

The implant according to the present invention is interposed between the proximal and the distal, segment cortices and thus is in position to transfer the load even if there is a significant mismatch in the contours of the segments rotated. The position of the grate-like implant is retained by at least 3 screws, preferably by four, two in each of the bone segments.

The shape of the grate-like implant is made cylindrical by bending so that the radius of the curvature closely matches the radius of curvature of the oscillating saw used to perform the osteotomy. The thickness of the implant is about the same as that of the saw.

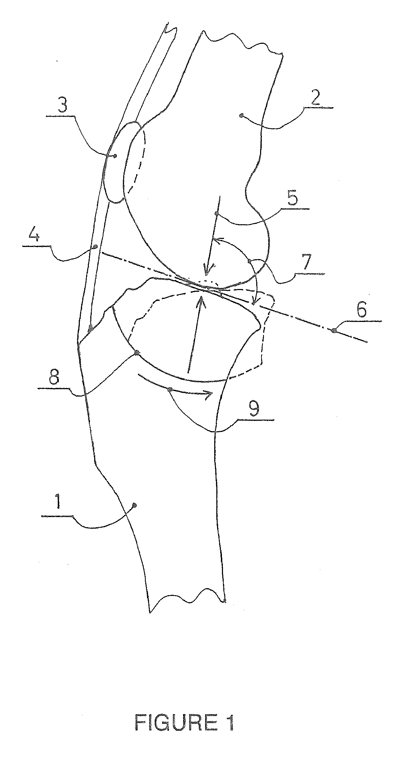

Figure 1 illustrates the knee joint both before and after the TPLO procedure. The tibia 1 and the femur 2 articulate at the knee joint via condyles of the femur and condyles of the tibia, divided by a plane, which is referred to as the common tangent 6. Pull from the quadriceps muscles acts on the patella 3, which in turn pulls the tibia via the patellar tendon 4. The force 5 between the condyles of the femur and the tibia is approximately parallel to the patellar tendon 4. When the joint force 5 is perpendicular to the common tangent 6, neither the cranial (anterior), nor the caudal (posterior) cruciate ligament are needed to stabilize the joint. When this condition is satisfied in the extended position of the knee, such as shown on Figure 1, the joint will also be stable when flexed, as stability is provided by the caudal (posterior) cruciate ligament.

However, in the normal stifle in extended position, the force 5 is not perpendicular to the common tangent 6, but the angle 7 is about 105 to 110 degrees, and the femur will tend to slide caudally off the tibia. To prevent this, a cylindrical osteotomy 8 is performed and the proximal segment of the tibia is rotated as indicated by arrow 9, usually by about 18 to 20 degrees. In the new position the angle between the force and the common tangent should be about 90, or a little less to guarantee joint stability.

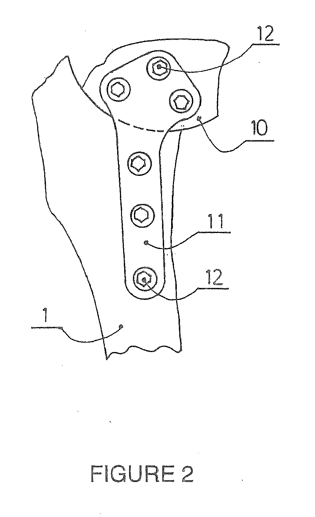

Figure 2 shows a conventional TPLO plate 11 as invented by Slocum. The plate is fixed to the tibia shaft 1 by screws 12 and to the proximal tibia segment 10 by screws 12.

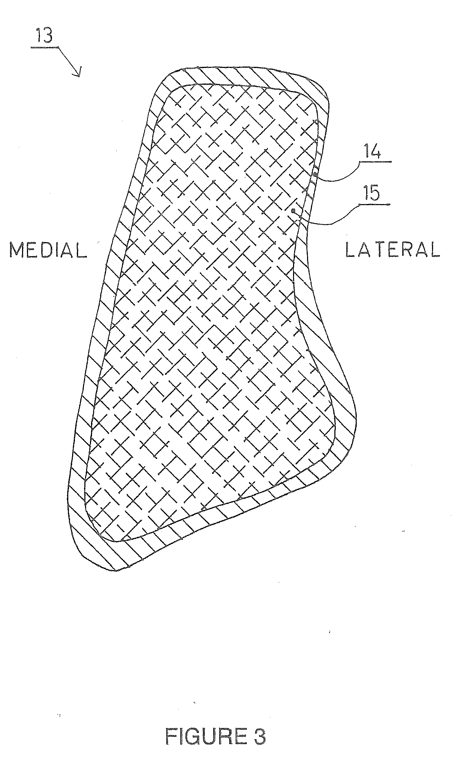

Figure 3 shows the shape of the bone section 13 along the cylindrical osteotomy for TPLO. Cancellous bone 15 is surrounded by the cortical bone 14. In some dogs the cancellous bone is very soft and weak at this level and most of the loading is to be taken by the cortical shell.

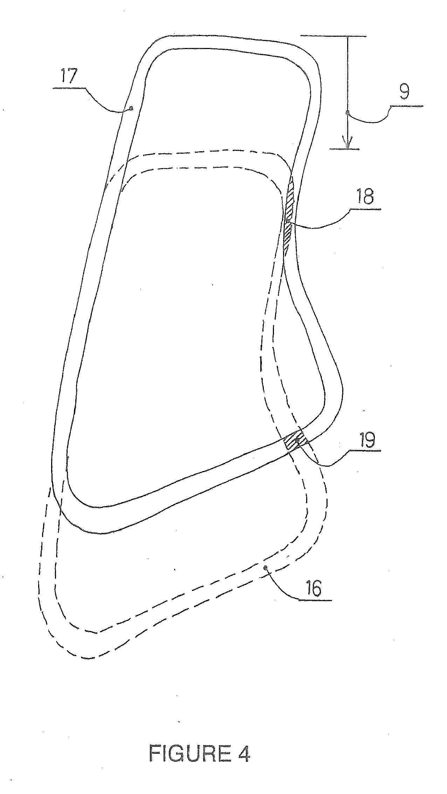

Figure 4 illustrates the poor contact between cortical shells of the proximal and distal segments once the shift 9 is performed. The medial cortices 17 still have a relatively good contact, but on the lateral side there are typically only two areas of contact 18 and 19 where the cortices cross each other.

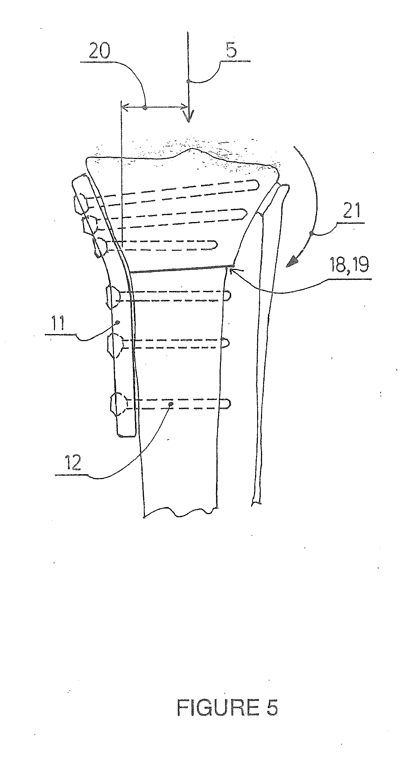

Figure 5 is a side, or frontal plane view of the conventional TPLO fixation by a medial plate 11 and screws 12. The joint force 5 is at a distance 20 from the plate 11 and the resulting bending moment 21 results in very high contact stresses at the lateral contact areas 18, 19. In many cases these stresses exceed the strength of the bone and lead to a partial collapse of the lateral support.

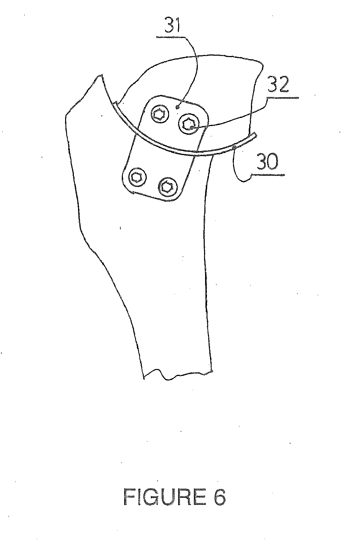

Figure 6 shows the TPLO fixation with an implant of the present invention. A thin spacer 30 is introduced into the gap created by the cylindrical saw and fixed to the tibia by a flange 31 and screws 32.

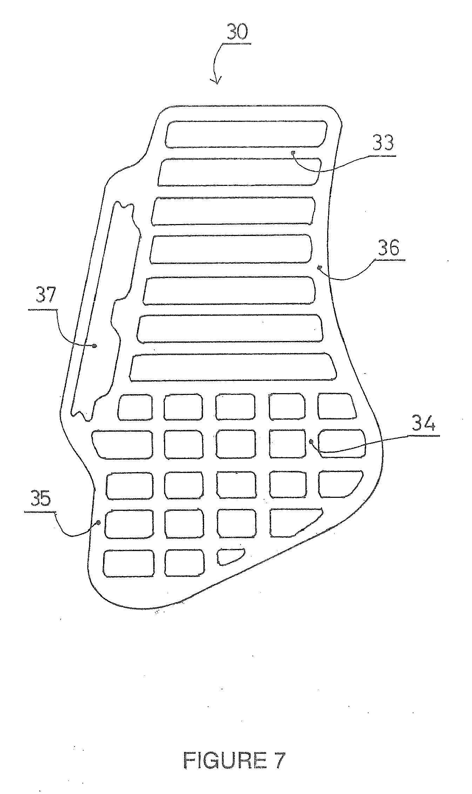

Figure 7 shows the grate-like spacer 30 as cut from thin sheet metal, most preferably c.p. titanium grade 4, with a plurality of ribs 33 connecting the medial 35 to the lateral side 36 of the implant. The caudal part of the implant also has a set of longitudinally running ribs 34 that provide extra stiffness to better support the overhanging part of the proximal tibia. On the medial side the grate-like spacer 30 is provided with a slit 37 to receive the flange 31 of the implant.

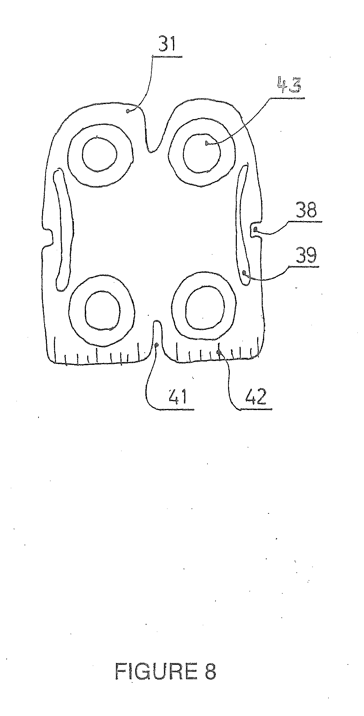

Figure 8 shows the flange 31 used to fix the grate 30 in position between the two bone segments, as well as to secure the rotation 9 of the proximal tibia segment 10. The flange is provided with holes 43 to receive the screws 32. The holes can be adopted for regular, non-locking screws, or they can be made to receive locking screws with conical heads. The recesses 38 are to engage the ends of the slit 37 in the grate 30. Slits 39 provide some flexibility to the sides of the flange 31, so that it can be snap-fitted into the slit 37 of the grate-like spacer 30.

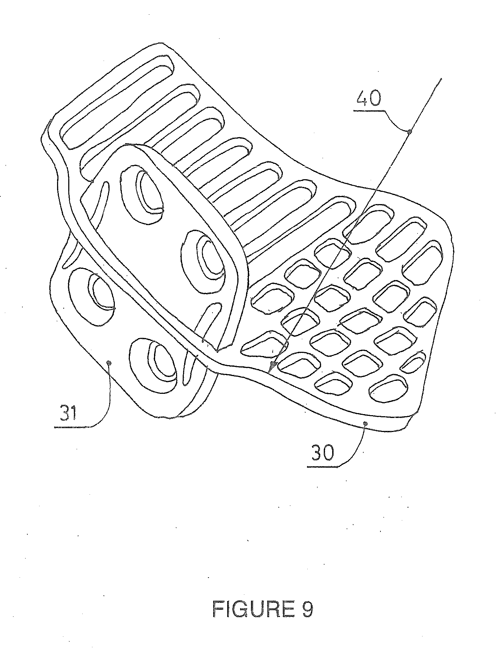

Figure 9 shows a perspective view of the implant according to the present invention assembled from the grate-like spacer 30 and the flange 31. The radius of curvature 40 is matched to the radius of curvature of the oscillating cylindrical saw used to perform the osteotomy.

The procedure of the present invention is very similar to the conventional TPLO in that the same main instrument - a cylindrical oscillating saw - is used to cut the osteotomy (i.e. to perform the curvilinear cut) and that the proximal segment of the tibia is rotated to level the plateau of the tibia. The main advantage is in the superior preservation of the bone tissue. Conventional TPLO plates produce vascular damage to the medial periosteum and consequently greatly increase the risks of infection, which has been reported as high as 10%.

Mechanical stability of the fixation according to the present invention is also a relevant improvement. Published reports on TPLO clinical outcomes suggest that as many as 15% of cases show partial collapse of the lateral support, which leads to unintended valgus deviation of the tibia. Total collapse of the lateral support can lead to very major complications with the proximal segment breaking into small pieces and thus very hard to fix in revision surgery.

There are also cost advantages to the implant system according to the present invention - lesser number of screws and a relatively simple main component - the grate-like spacer and the flange can be efficiently produced from sheet metal by e.g laser cutting or water jet cutting.

Surgery time is also reduced. Markings 41 along the distal edge of the flange can be produced to indicate the angular inclination. Once inserted into the saw gap, the implant is first fixed to the proximal segment of the tibia and it can then be used to effect the rotation before the fixation to the distal tibia segment. The scale can be read against a mark made on the distal fragment of the tibia adjacent to the flange edge. This is routinely done by an osteotome. In the conventional surgical procedure a large pin is driven into the proximal segment to give a handle by which the segment is rotated and fixed in position by yet another K-wire inserted through the distal into the proximal segment of the tibia. These additional holes and pins produce damage to the fragile proximal segment and also take time to execute.

The basic function of the implant according to the present invention is to facilitate transfer of compression between the segments of the tibia across the osteotomy, while allowing the bone to grow through the grate-like spacer with minimal impediment.

Bone conductive or bone inductive surface treatments are desired, but optional. If the metal of the cage is titanium, or an alloy of titanium, surface treatments can be one of: pickling, anodizing, glow discharge anodizing with addition of calcium phosphate, plasma coating with porous titanium or with hydroxyapatite.

Preferred range for the thickness of the grate-like spacer is 0.7 to 1.6 mm, most preferably 1 mm. Preferred range for the thickness of the flange is the same as that for the spacer. The radius of curvature is the same as that of the cylindrical saws used for TPLO, currently available in increments of 2 to 3mm, from 10 mm to 33 mm.

The surface finish of the spacer can include rough blasting to increase friction and reduce gliding of the bone segments over the implant.

The procedure of the present invention includes rotation of the tibial plateau according to TPLO of Slocum, but by the amount that brings the common tangent to 90 degrees with respect to the patellar tendon with the stifle extended.

Occasionally TPLO surgery performed for cruciate rupture is also combined with surgical intervention to correct tibia shape that may be responsible for overall mal-alignment of the limb. If done, this is most often to correct for varus deformity and internal torsion of the tibia. This calls for either multiple cuts at the osteotomy site, or it can be done with e.g. locking TPLO plates that can tolerate some gaps at the osteotomy. The implant and the method of use disclosed here are not addressing the need for these measures. However, the grate-like implant can be produced to allow for these corrections without leaving undue gaps at the osteotomy. Most relevant is making the implant wedge shaped with its thickness being larger on the medial side, resulting in the effect equivalent to medial open wedge osteotomy. More complex, but possible, is the use of the wedge shaped implant according to the present invention with larger thickness on the lateral side.

Moreover, an implant with a shape conforming to the bone segments placed after osteotomy into relative rotation to each other can be produced. Such implants can be manufactured as single pieces, or can be assembled from multiple components.

Having disclosed at least one embodiment of the present invention, various adaptations, modifications, additions, and improvements will be readily apparent to those of ordinary skill in the art. Such adaptations, modifications, additions and improvements are considered part of the invention which is only limited by the several claims attached hereto.

## Claims

1. An implant for fixing two segments of a bone that are produced in an osteotomy by a curvilinear cut, the implant comprising
(i) a curved plate (30), adapted to be interposed between the two segments of the bone, and
(ii) a flange-like means (31), adapted to allow for its fixation to the two bone segments.

2. The implant of claim 1, wherein the curved plate (30) has a curvature corresponding to the curvilinear cut separating the two segments of the bone.

3. The implant of claim 2, wherein the radius of curvature (40) of the curved plate (30) is the same as that of the cylindrical saw used for the cut, e.g. in the range of 5 to 50 mm, particularly 10 to 33 mm or 12 to 20 mm.

4. The implant of any one of the preceding claims, wherein the shape of the curved plate (30) corresponds to the shape of the cross section of the two segments of a bone produced by the curvilinear cut, comprising a cranial section and a caudal section.

5. The implant of any one of the preceding claims, wherein the curved plate (30) is at least partially perforated, preferably in a grate-like manner.

6. The implant of claim 5, wherein the curved plate (30) comprises slits and/or holes.

7. The implant of claim 6, wherein the cranial part of the curved plate (30) is provided with slits and/or the caudal part of the curved plate (30) is provided with holes.

8. The implant of claim 7, wherein the caudal part of the curved plate (30) is perforated in a grate-like manner, comprising a set of ribs (33) connecting the medial side (35) to the lateral side (36) of the curved plate (30) and a set of longitudinally running ribs (34) to provide extra stiffness.

9. The implant of any one of the preceding claims, wherein the thickness of the curved plate (30) corresponds to the gap produced by the cut separating the two segments of the bone, particularly in the range of 0.7 to 1.6 mm, preferably 0,8 to1.2 mm, e.g. about 1 mm.

10. The implant of any one of the preceding claims, wherein the thickness of the curved plate (30) is larger on the medial side (35) than on the lateral side (36).

11. The implant of any one of claims 1-9, wherein the thickness of the curved plate (30) is larger on the lateral side (36) than on the medial side (35).

12. The implant of any one of the preceding claims, comprising an artificial biocompatible material selected from the group consisting of titanium, titanium alloy, stainless steel and cobalt alloy.

13. The implant of any one of the preceding claims, wherein the surface of the curved plate (30) and/or the flange-like means (31) is treated for optimum bone tissue tolerance and/or adhesion.

14. The implant of any one of the preceding claims, wherein the surface of the curved plate (30) and/or the flange-like means (31) is configured to provide high friction and/or mechanical interlock and/or to favor intimate bone apposition or ingrowth.

15. The implant of any one of the preceding claims, wherein the curved plate (30) is provided with a slit (37) to receive the flange-like means (31).

16. The implant of claim 15, wherein the slit is located at the medial side (35) and/or at the lateral side (36) of the curved plate (30).

17. The implant of any one of the preceding claims, wherein the flange-like means (31) is provided with at least one recess (38) to engage the ends of the slit (37).

18. The implant of any one of the preceding claims, wherein the flange-like means (31) is provided with at least one slit (39) configured to provide flexibility to the sides of the flange-like means (31).

19. The implant of any one of the preceding claims, wherein the flange-like means (31) is provided with at least two, preferably at least four holes (43) adopted to receive screws (32).

20. The implant of any any one of the preceding claims, wherein the flange-like means (31) is provided with a scale (42).

21. The implant of any one of the preceding claims, wherein the curved plate (30) is manufactured as a single piece or assembled from multiple components.

22. The implant of any one of the preceding claims, wherein the shape of the curved plate (30) is conform to the bone segments placed after osteotomy into relative rotation to each other.

23. An orthopedic method, comprising the step of fixing two bone segments using an implant of any one of claims 1-22.

24. The method of claim 23, comprising the steps
(i) making a curvilinear cut in a bone, e.g. a left or right tibia, to define two bone segments,
(ii) interposing a curved plate (30) as defined in any one of claims 1-22 between the two bone segments, and
(iii) fixing the curved plate to the two bone segments via a flange-like means (31).
